# EUROPEAN PATENT APPLICATION

(11) **EP 4 303 305 A1**
(43) Date of publication of application: **10.01.2024**
(21) Application number: 22183641.4
(22) Date of filing: 07.07.2022
(51) Int. Cl.: C12N 15/62

(54) **FUSION POLYPEPTIDES FOR CELL PENETRATION AND CELL TARGETING**

(71) Applicant: Kutzner, Christoph, 68219 Mannheim (DE)
(72) Inventor: Kutzner, Christoph, 68219 Mannheim (DE)
(74) Representative: Eisenführ Speiser

(57) **Abstract**

The present invention concerns fusion polypeptides comprising a cell penetration and a substrate binding domain. Additionally, the present invention concerns a genetically modified cell expressing these fusion polypeptides as well as the use of these fusion polypeptides for introducing a substance into a target cell.

## Description

The present invention concerns fusion polypeptides comprising a cell penetration and a substrate binding domain. Additionally, the present invention concerns a genetically modified cell expressing these fusion polypeptides as well as the use of these fusion polypeptides for introducing a substance into a target cell.

Delivering substances to the cell is the basis for e.g. a successful drug-targeting or specific epigenetic modification. For this purpose active pharmaceutical ingredients or DNA/RNA sequences must be introduced into a target cell. To achieve an introduction of a sequence into a cell, the cell barrier must be passed, which protects the cell against environmental influences. Besides the protection from environmental influences, the cell requires certain substances and signal molecules from other cells of the organism, which need to have access to the cell.

There are several mechanism for mass transport in and out of a cell for retaining the natural balance and provide the required substances to a cell. The independent and undirected mass transport ways into the cell are diffusion and osmosis. These processes do not require energy and are thus called passive mass transport. Active mass transport requires energy. Such active transport processes are e.g. membrane proteins, endocytosis and exocytosis.

The natural transport processes inside the cell can be targeted to deliver active substances, such as e.g. pharmaceuticals to a cancer cell or genetic information for gene silencing.

Another way of introducing substances into a cell is the use of cell-penetrating peptides (CPP). These peptides allow an energy-independent pathway for delivering substances inside a cell, which were thought to be only deliverable by nearly energy-dependent pathways. CPPs are associated with the substances, which are thought to be introduced into a cell ("cargo"), either through covalent bonds or non-covalent interactions. CPPs can be of different sizes, amino acid sequences, and charges, but all CPPs have the ability to translocate the plasma membrane and facilitate the delivery of various molecular cargoes to the cytoplasm or an organelle.

CPPs are specific for certain organisms or cell-types depending on the cell membrane composition. Plant cells require different CPPs than mammalian cells for introducing the cargo into the cell. Numata et al. ("Library screening of cell-penetrating peptide for BY-2 cells, leaves of Arabidopsis, tobacco, tomato, poplar and rice callus", (2018)8:10966, Nature scientific reports*)* report the screening of different CPPs for targeting different plant species. It was immanent that the results for the different CPPs vary strongly between the different target plants and are dependent on the secondary structure, the CPPs are forming.

One big challenges in utilizing CPPs is the dependency of the transport efficiency on the cargo. It would be preferred if CPPs that were identified to have a good transport efficiency can be used for a wide variety of different cargo, e.g. transporting polypeptides as well as small molecules or DNA/RNA.

CPPs, especially cationic peptides, are assumed to enable the membrane passing of fusion polypeptides by non-endosomal mechanisms. The CPPs Tat and Arg9 were compared to find their ability to transduce polypeptides but also nucleic acids and it was found that a large portion of the CPP labelled cargo was simply adhered to the cell surface and not transported into cell, while nucleic acid cargo was introduced *("*Cell-penetrating peptides - A Reevaluation Of The Mechanism Of Cellular Uptake", Richard et al., The Journal of biological Chemistry, 278(1) (585-590), 2003*).*

It is thus desirable to obtain CPPs, which can be used for different types of cargo without screening for each type of cell and each type of cargo the optimal.

Another drawback in the utilization of CPPs for cargo delivery into cells is that cell penetrating peptides as transport molecules are difficult to be produced in the amounts for achieving the concentrations that are needed for delivering an appropriate amount of cargo into the cell. Furthermore, the applications for CPP-mediated transport are limited due to high molecular weight and high amount of required negative charges. The uptake of cargo via CPP-mediated transport is low compared to the high amount of peptide needed. Fusion polypeptides could be a way to overcome this low efficiency problem, but covalent linkage of the cargo could lead to an inefficient effect of the substance in the cell.

Thus, the primary object of the present invention was to provide a tool for targeted delivery of cargo inside a cell, which is applicable for a variety of different cell types in different organisms and can be used with different types of cargo.

Another object of the present invention was to provide such tool, which can be produced economically and easy to scale-up.

The primary object of the present invention was solved by providing a fusion polypeptide comprising or consisting of at least three functional domains:
i) a substrate binding domain binding to a substrate non-covalently, wherein the substrate is selected from the group consisting of polypeptides, polysaccharides, DNA, RNA or a mixture of DNA and RNA ;
ii) a cell penetrating domain;
iii) a signal sequence;
iv) optionally a linker domain; and
v) optionally a pH-sensitive autoprotease domain,
   wherein the cell penetrating domain has a length of between 15 and 50 amino acid residues, preferably of 20 to 40 amino acid residues and especially preferably of between 24 to 34 amino acid residues, and
   wherein the cell penetrating domain comprises at least 70 %, preferably at least 75 % and especially preferably 80 % amino acid residues with a helix potential value >1, dependent on the overall length of the cell penetrating domain.

The substrate binding domain of the fusion polypeptide according to the invention receives the substrate or cargo, which should be introduced into the cell. The fusion polypeptide according to the invention is brought into contact with the cargo and the cargo binds non-covalently to the substrate binding domain.

It was discovered that besides the receiving of the cargo, which should be introduced into the cell, the substrate binding domain plays also a role in target delivery of the cargo inside the cell, as it can also bind to cellular structures such as DNA/RNA or the actin cytoskeleton.

The cell penetrating domain of the fusion polypeptide according to the invention allows the cargo to be introduced into the cell by triggering the membrane translocation mechanism. Preferably, the cell penetrating domain of the fusion polypeptide according to the invention triggers the endocytosis-mediated translocation or translocation through the formation of a transitory structure.

It was surprisingly found that the use of a cell penetrating domain having a length of between 15 and 50 amino acid residues, preferably of 20 to 40 amino acid residues and especially preferably of between 24 to 34 amino acid residues, and comprising at least 70 %, preferably at least 75 % and especially preferably 80 % amino acid residues with a helix potential value >1, dependent on the overall length of the cell penetrating domain, allow a high efficiency of introduced cargo into the cell as well as the compatibility with a variety of different organisms.

The helix potential (Pα) is the determined value for the frequency of amino acids to be incorporated into α-helical secondary structures. Amino acids having a Pa value of >1 are helical forming amino acids, whereas amino acids having a Pa value of <1 are helical disrupting amino acids. Table 1 shows an overview of the helix potentials of proteinogenic amino acids according to Stryer Biochemie, 5th edition, page 73, Table 3.3.

The cell penetrating domain according to the invention thus forms a compact stable secondary helical structure, which leads to an overall improved cell penetrating performance.

**Table 1: Overview of amino acids and their helix potential**

| Amino acid | Helix potential (Pα) |
|---|---|
| Glutamic acid (Glu) | 1.59 |
| Alanine (Ala) | 1.41 |
| Leucine (Leu) | 1.34 |
| Methionine (Met) | 1.30 |
| Glutamine (Gln) | 1.27 |
| Lysine (Lys) | 1.23 |
| Arginine (Arg) | 1.21 |
| Phenylalanine (Phe) | 1.16 |
| Isoleucine (Ile) | 1.09 |
| Histidine (His) | 1.05 |
| Tryptophan (Trp) | 1.02 |
| Aspartic acid (Asp) | 0.99 |
| Valine (Val) | 0.90 |
| Threonine (Thr) | 0.76 |
| Asparagine (Asn) | 0.76 |
| Cystein (Cys) | 0.66 |
| Tyrosine (Tyr) | 0.61 |
| Serine (Ser) | 0.57 |
| Glycine (Gly) | 0.43 |
| Proline (Pro) | 0.34 |

The signal sequence of the fusion polypeptide according to the invention allows the fusion polypeptide to be guided to certain cell compartments. This is beneficial either in the production of these fusion polypeptides in recombinant expression systems as well as in the cellular targeting of the cargo bound to the substrate binding domain of the fusion polypeptide.

Preferably, the signal sequence of the fusion polypeptide according to the invention is an inclusion body promoting sequence or a secretion sequence, preferably of secretion type IV or type II of gram negative bacteria. These sequences allow the production of the fusion polypeptide according to the invention in gram-negative recombinant production systems, such as e.g. *E.coli.*

Moreover preferably, the signal sequence of the fusion polypeptide according to the invention is a commonly known signal sequence, e.g. as described in Stryer Biochemie, 5. Auflage, table 12.4.

Preferably, the present invention relates to a fusion polypeptide according to the invention, wherein the cell penetrating domain comprises or consists of at least one amino acid sequence selected from the group consisting of SEQ ID NOs.: 1 to 24, or an amino acid sequence having a sequence identity of more than 90 %, preferably at least 91 %, preferably at least 92 %, preferably at least 93 %, preferably at least 94 %, preferably at least 95 %, preferably at least 96 %, preferably at least 97 %, preferably at least 98 % preferably at least 99 % to an amino acid sequence selected from the group consisting of SEQ ID NOs.: 1 to 24.

Especially preferably, the present invention relates to a fusion polypeptide according to the invention, wherein the cell penetrating domain comprises or consists of at least one amino acid sequence selected from the group consisting of SEQ ID NOs.: 1, 2, 3, 17, 18, 19, 20 and 21or an amino acid sequence having a sequence identity of more than 90 %, preferably at least 91 %, preferably at least 92 %, preferably at least 93 %, preferably at least 94 %, preferably at least 95 %, preferably at least 96 %, preferably at least 97 %, preferably at least 98 % preferably at least 99 % to an amino acid sequence selected from the group consisting of SEQ ID NOs.: 1, 2, 3, 17, 18, 19, 20 and 21.

Whenever the present disclosure relates to the percentage of identity of nucleic acid or amino acid sequences to each other these values define those values as obtained by using the EMBOSS Water Pairwise Sequence Alignments (nucleotide) program or the EMBOSS Water Pairwise Sequence Alignments (polypeptide) program for amino acid sequences. Alignments or sequence comparisons as used herein refer to an alignment over the whole length of two sequences compared to each other. Those tools provided by the European Molecular Biology Laboratory (EMBL) European Bioinformatics Institute (EBI) for local sequence alignments use a modified Smith-Waterman algorithm (see Smith, T.F. & Waterman, M.S. "Identification of common molecular subsequences" Journal of Molecular Biology, 1981 147 (1):195-197). When conducting an alignment, the default parameters defined by the EMBL-EBI are used. Those parameters are (i) for amino acid sequences: Matrix = BLOSUM62, gap open penalty = 10 and gap extend penalty = 0.5 or (ii) for nucleic acid sequences: Matrix = DNAfull, gap open penalty = 10 and gap extend penalty = 0.5. The skilled person is well aware of the fact that, for example, a sequence encoding a polypeptide can be "codon-optimized" if the respective sequence is to be used in another organism in comparison to the original organism a molecule originates from.

Preferably, the present invention relates to a fusion polypeptide according to the invention, wherein the substrate binding domain comprises at least one sequence of the amino acid motif sequences IHYWN and VYVKV.

The preferred amino acid motif sequences in terms of the present invention were found to have a significant influence on the binding properties of the substrate binding domain and can be found in all substrate binding domains according to the invention.

Furthermore, it is preferred if two, three, four, five or more repetitions of these two amino acid motif sequences are present in the substrate binding domain according to the invention. Preferably, the repetitions are incorporated in the substrate binding domain in a consecutive order or separated by short sequences with hydrophobic amino acids. It is preferred in terms of the present invention if the short sequences with hydrophobic amino acids have a maximum length of 20 amino acids, wherein at least 50 %, preferably 60 %, especially preferably 70 % amino acids dependent on the short sequence length are hydrophobic amino acids.

Hydrophobic amino acids in terms of the present invention are amino acids selected from the group consisting of alanine, valine, leucine, isoleucine, phenylalanine, proline, tryptophan and methionine.

Preferably, the present invention relates also to a fusion polypeptide according to the present invention, wherein the substrate binding domain comprises or consist of an amino acid sequence having a length of between 200 and 600, preferably of between 250 and 550 amino acids.

Moreover preferably, the present invention relates to a fusion polypeptide according to the invention, wherein the substrate binding domain comprises or consists of at least one amino acid sequence selected from the group consisting of SEQ ID Nos.: 25 and 26 or an amino acid sequence having at least 90 %, preferably at least 91 %, preferably at least 92 %, preferably at least 93 %, preferably at least 94 %, preferably at least 95 %, preferably at least 96 %, preferably at least 97 %, preferably at least 98 % preferably at least 99 % sequence identity to an amino acid sequence selected from the group consisting of SEQ ID Nos.: 25 and 26.

Preferably, the present invention relates to a fusion polypeptide according to the invention, wherein the signal sequence is an inclusion body promoting sequence or a secretion sequence, preferably a secretion sequence of secretion type IV or type II of gram negative bacteria.

Moreover preferably, the present invention relates to a fusion polypeptide according to the invention, wherein the signal sequence comprises or consists of at least one amino acid sequence selected from the group consisting of SEQ ID Nos.: 27 and 28 or an amino acid sequence having at least 90 %, preferably at least 91 %, preferably at least 92 %, preferably at least 93 %, preferably at least 94 %, preferably at least 95 %, preferably at least 96 %, preferably at least 97 %, preferably at least 98 % preferably at least 99 % sequence identity to an amino acid sequence selected from the group consisting of SEQ ID Nos.: 27 and 28.

Preferably, the fusion polypeptide according to the invention comprises a linker domain and preferably, the linker domain comprises an N-terminal alpha helix and/or a C-terminal sequence of a random coil structure, preferably wherein the linker domain comprises or consists of at least one amino acid sequence selected from the group consisting of SEQ ID Nos.:29 and 30 or an amino acid sequence having at least 90 %, preferably at least 91 %, preferably at least 92 %, preferably at least 93 %, preferably at least 94 %, preferably at least 95 %, preferably at least 96 %, preferably at least 97 %, preferably at least 98 % or preferably at least 99 % sequence identity to an amino acid sequence selected from the group consisting of SEQ ID Nos.:29 and 30.

A linker sequence in the context of the present invention means a sequence between the functional domains. The length of the linker is preferably 1 to 50 or more than 50 amino acids.

Preferably, the present invention relates to a fusion polypeptide according to the present invention, wherein the substrate to which the substrate binding domain binds non-covalently is DNA or RNA having a length of 3 to 3,000,000 base pairs, preferably a length of 3,000 to 30,000 base pairs, especially preferably a length of 30,000 to 300,000 base pairs.

Also preferably, the substrate binding domain of the fusion polypeptide according to the invention binds polypeptides having an amino acid sequence length of 5 to 250 amino acids, preferably 20 to 200 amino acids and especially preferably 50 to 150 amino acids.

Moreover preferably, the substrate binding domain of the fusion polypeptide according to the invention binds to a polypeptide, which comprises a polysaccharide tag having a minimal length of a disaccharide, preferably an amylopectin or dextrin tag.

Preferably, the present invention relates to a fusion polypeptide according to the invention, wherein the fusion polypeptide comprises or consists of an amino acid sequence selected from the group consisting of SEQ ID Nos.: 32 to 99 or an amino acid sequence having at least 90 %, preferably at least 91 %, preferably at least 92 %, preferably at least 93 %, preferably at least 94 %, preferably at least 95 %, preferably at least 96 %, preferably at least 97 %, preferably at least 98 % preferably at least 99 % sequence identity to an amino acid sequence selected from the group consisting of SEQ ID Nos.: 32 to 99.

The SEQ ID NOs.: 32 to 99 are sequences of fusion polypeptides having the functional domains according to the invention. These fusion polypeptides are suitable for transferring a wide variety of different substrates into a cell.

Preferably, the substrates are selected from the group consisting of antibodies such as bermekimab or clazakizumab and polysaccharide tagged bermekimab or clazakizumab, small molecules such as adamantane or fullerene and polysaccharide tagged adamantane or fullerene, antibiotics such as chloramphenicol or kanamycin and polysaccharaide tagged chloramphenicol and kanamycin, biologics such as insulin or human growth factor and polysaccharide tagged insulin or human growth factor, polypeptides such as melatonin or lipocalin and polysaccharide tagged melatonin or lipocalin, transcription factors such as e.g. zinc finger polypeptides, DNA (such as e.g. plasmid DNA, viral double stranded DNA or linear cDNA), RNA (such as e.g. siRNA, mRNA, or double stranded RNA ), or a mixture of DNA and RNA.

Preferably, the present invention relates to a fusion polypeptide, wherein the fusion polypeptide comprises or consists of the five functional domains i) to v), i.e. domains iv) and v) are present and not optional in this case,

preferably wherein the pH-sensitive autoprotease domain comprises or consists of at least one amino acid sequence according to SEQ ID No.: 31 or an amino acid sequence having at least 90 %, preferably at least 91 %, preferably at least 92 %, preferably at least 93 %, preferably at least 94 %, preferably at least 95 %, preferably at least 96 %, preferably at least 97 %, preferably at least 98 % preferably at least 99 % sequence identity to an amino acid sequence according to SEQ ID No.: 31.

The pH-sensitive autoprotease is capable of releasing the peptide as a response to the pH-value within the cell. Preferably, the pH-sensitive autoprotease is active at a pH-value of between 6.8 and 7.2.

Another aspect of the present invention relates to a recombinant nucleic acid molecule encoding a fusion polypeptide according to the invention.

Preferably, the cell penetrating peptide domain of the fusion polypeptide according to the invention is encoded by a nucleic acid molecule selected from the group consisting of SEQ ID NOs.: 100 to 123 or a nucleic acid molecule having at least 90 %, preferably at least 91 %, preferably at least 92 %, preferably at least 93 %, preferably at least 94 %, preferably at least 95 %, preferably at least 96 %, preferably at least 97 %, preferably at least 98 % preferably at least 99 % sequence identity to a nucleic acid molecule selected from the group consisting of SEQ ID NOs.: 100 to 123.

Preferably, the substrate binding domain of the fusion polypeptide according to the invention is encoded by a nucleic acid molecule selected from the group consisting of SEQ ID NOs.: 124 and 125 or a nucleic acid molecule having at least 90 %, preferably at least 91 %, preferably at least 92 %, preferably at least 93 %, preferably at least 94 %, preferably at least 95 %, preferably at least 96 %, preferably at least 97 %, preferably at least 98 % preferably at least 99 % sequence identity to a nucleic acid molecule selected from the group consisting of SEQ ID NOs.: 124 and 125.

Preferably, the signal sequence domain of the fusion polypeptide according to the invention is encoded by a nucleic acid molecule selected from the group consisting of SEQ ID NOs.: 126 and 127 or a nucleic acid molecule having at least 90 %, preferably at least 91 %, preferably at least 92 %, preferably at least 93 %, preferably at least 94 %, preferably at least 95 %, preferably at least 96 %, preferably at least 97 %, preferably at least 98 % preferably at least 99 % sequence identity to a nucleic acid molecule selected from the group consisting of SEQ ID NOs.: 126 and 127.

Preferably, the fusion polypeptide according to the invention comprises a linker domain, which is encoded by a nucleic acid molecule selected from the group consisting of SEQ ID NOs.: 128 and 129 or a nucleic acid molecule having at least 90 %, preferably at least 91 %, preferably at least 92 %, preferably at least 93 %, preferably at least 94 %, preferably at least 95 %, preferably at least 96 %, preferably at least 97 %, preferably at least 98 % preferably at least 99 % sequence identity to a nucleic acid molecule selected from the group consisting of SEQ ID NOs.: 128 and 129.

Preferably, the fusion polypeptide according to the invention comprises a pH-sensitive autoprotease domain, which is encoded by a nucleic acid molecule according to SEQ ID NO.: 130 or a nucleic acid molecule having at least 90 %, preferably at least 91 %, preferably at least 92 %, preferably at least 93 %, preferably at least 94 %, preferably at least 95 %, preferably at least 96 %, preferably at least 97 %, preferably at least 98 % preferably at least 99 % sequence identity to a nucleic acid molecule according to SEQ ID NO.: 130.

Preferably, the fusion polypeptide according to the invention is encoded by a nucleic acid molecule selected from the group consisting of SEQ ID NOs.: 131 to 250 or a nucleic acid molecule having at least 90 %, preferably at least 91 %, preferably at least 92 %, preferably at least 93 %, preferably at least 94 %, preferably at least 95 %, preferably at least 96 %, preferably at least 97 %, preferably at least 98 % preferably at least 99 % sequence identity to a nucleic acid molecule selected from the group consisting of SEQ ID NOs.: 131 to 250.

Another aspect of the present invention relates to a genetically modified cell, including a recombinant nucleic acid molecule according to the invention, wherein the cell is capable of expressing a fusion polypeptide according to the invention, preferably wherein the cell is selected from the group consisting of *Escherichia coli, Vibrio natrigens, Saccheromyces cerevisiae, Aspergillus niger,* green algae, microalgae, HEK T293 and Chinese hamster ovary cells (CHO).

Yet another aspect of the present invention relates to a method for producing a fusion polypeptide according to the invention, comprising the steps of
a) providing a genetically modified cell according to the invention;
b) culturing the cell under conditions suitable for expression of a fusion polypeptide according to the invention;
c) obtaining the fusion polypeptide and optionally, unfolding of the obtained fusion polypeptide and directed refolding of said fusion polypeptide.

Step (a) comprises providing a genetically modified cell expressing a fusion polypeptide. Such cell is obtainable by introducing a nucleic acid molecule including a sequence encoding a fusion polypeptide according to the invention, preferably in the form of a vector, into the cell by known methods such as for example by transfection or transformation.

In step (b), the cell is cultured under conditions suitable for expressing a fusion polypeptide according to the invention, preferably in a high-density culture. Culture conditions and especially conditions to achieve a high-density culture and corresponding media are well known to the person skilled in the art. Preferably, the expression of the fusion polypeptide is achieved with a subsequent transport to inclusion bodies using a suitable signal sequence according to the invention.

Step (c) comprises obtaining the fusion polypeptide from the culture broth and optionally unfolding of the obtained fusion polypeptide and directed refolding of said fusion polypeptide, if the fusion polypeptide is present in inclusion bodies. Solubilization conditions for the processing of inclusion bodies and conditions for the directed refolding are well known in the art. Preferably, inclusion bodies are solubilized by using 6 M guanidinium chloride, 8 M urea or 2 % sodium dodecyl sulfate and are refolded under neutral or mildly basic conditions.

One aspect of the present invention relates to a method for introducing a substrate into a cell, comprising the steps of
a) providing a fusion polypeptide according to the invention, preferably obtained by a method according to the invention;
b) contacting the fusion polypeptide according to the invention with the substrate, which should be introduced into the cell;
c) obtaining a fusion polypeptide with a substrate, which is bond non-covalently to the substrate binding domain of the fusion polypeptide;
d) contacting the obtained fusion polypeptide / substrate complex obtained in step c) with the cell, in which the substrate should be introduced.
e) obtaining a cell with an introduced substrate.

Preferably, the method is performed ex *vivo.*

Preferably, the step of contacting b) is performed at a temperature of 20 to 37 °C and a pH value of between 6 and 9 in the presence of a buffer selected from TRIS, HEPES, PIPES or a buffer containing sodium acetate or arginine and sucrose.

Also preferably, the contacting step d) is done using the low pressure spray method (LPS).

Another aspect of the present invention related to a non-therapeutic use of a fusion polypeptide according to the invention for introducing a substance into a target cell, wherein the substance is selected from the group consisting of antibodies, small molecules, antibiotics, biologics, polypeptides, transcription factors or DNA, RNA or a mixture of DNA and RNA.

Preferably, the substrate is selected from the group consisting of antibodies such as bermekimab or clazakizumab and polysaccharide tagged bermekimab or clazakizumab, small molecules such as adamantane or fullerene and polysaccharide tagged adamantane or fullerene, antibiotics such as chloramphenicol or kanamycin and polysaccharaide tagged chloramphenicol and kanamycin, biologics such as insulin or human growth factor and polysaccharide tagged insulin or human growth factor, polypeptides such as melatonin or lipocalin and polysaccharide tagged melatonin or lipocalin, transcription factors such as e.g. zinc finger polypeptides, DNA (such as e.g. plasmid DNA, viral double stranded DNA or linear cDNA), RNA (such as e.g. siRNA, mRNA, or double stranded RNA ), or a mixture of DNA and RNA.

Another aspect of the invention relates to the non-therapeutic use of a fusion polypeptide according to the invention, wherein the target cell is selected from the group consisting of plant cells, bacterial cells, mammalian cells and yeast cells, preferably plant cells.

Further aspects and advantages of the invention result from the subsequent description of preferred examples.

### Short description of sequences

SEQ ID NOs.: 1 to SEQ ID NO. 24 describe amino acid sequences encoding the artificial cell penetrating peptide 1 to 24.
SEQ ID NOs.: 25 and 26 describe amino acid sequences encoding binding domains.
SEQ ID NOs.: 27 and 28 describe amino acid sequences encoding signal sequences.
SEQ ID NO.: 29 and 30 describe amino acid sequences encoding linker domains.
SEQ ID NO.: 31 describes an amino acid sequence encoding a pH-sensitive autoprotease.
SEQ ID NOs.: 32 to 99 describe amino acid sequences encoding fusion polypeptides according to the invention.
SEQ ID NOs.: 100 to 123 describe nucleic acid sequences encoding the artificial cell penetrating peptide 1 to 24.
SEQ ID NOs.: 124 and 125 describe nucleic acid sequences encoding binding domains.
SEQ ID NOs.: 126 and 127 describe nucleic acid sequences encoding linker domains.
SEQ ID NOs.: 128 and 129 describe nucleic acid sequences encoding linker domains.
SEQ ID NO.: 130 describes a nucleic acid domain encoding a pH-sensitive autoprotease.
SEQ ID No.: 131 to 250 describe nucleic acid sequences encoding fusion polypeptides according to the invention.

### Short description of Figures

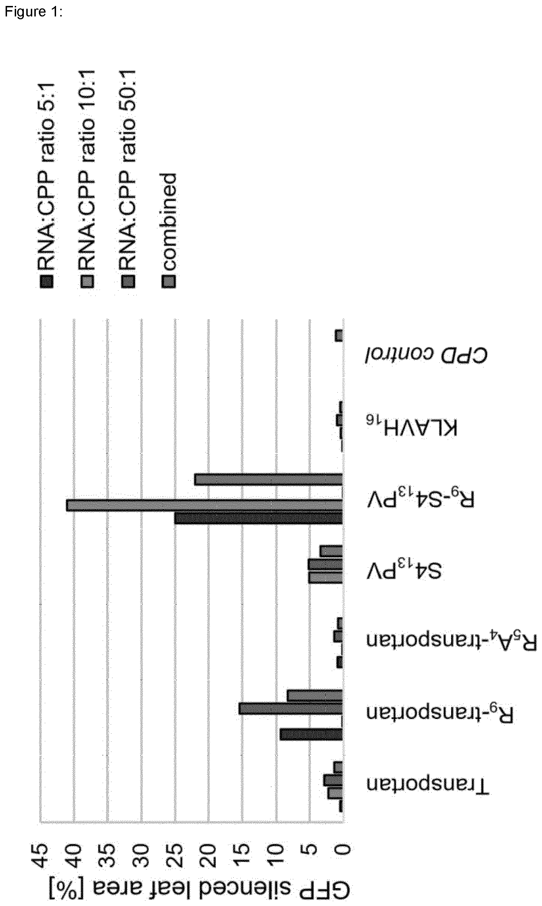

Figure 1: Graph showing the mean value of the GFP silencing ratio of all silenced leaves originating from the treated strain apical meristem with regard to the total leaf area. The results of LPS treatment with transportan-, Rg-transportan-, R₅A₄-transportan-, S4₁₃PV-, R₉-S4₁₃PV- and KLAVH₁₆- 22nt siRNA complex in the molar RNA:CPP ratios 5:1, 10:1, 50:1, as well as the overall result (combined) are shown. The 10 % CPD-22nt siRNA complex control is shown under the category 'combined'. The number of leaves evaluated depends on the individual and ranges from 1 to 6.

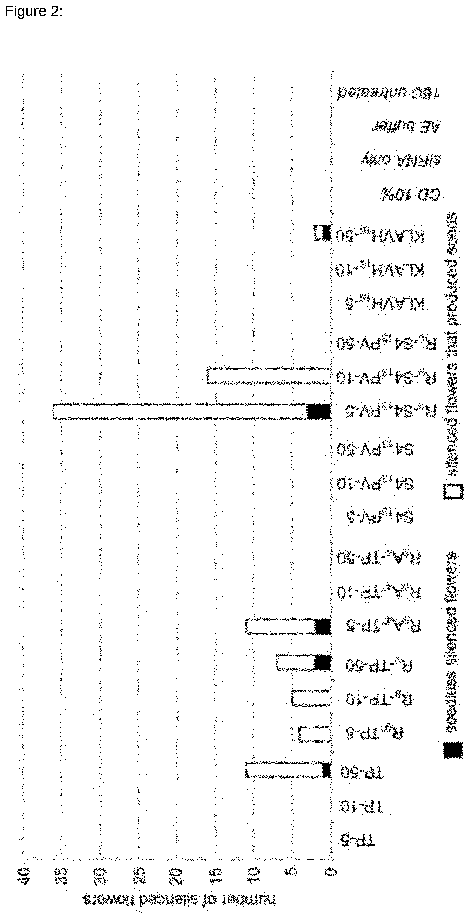

Figure 2: Number of GFP silenced flowers developed by CPP-22nt siRNA complex LPS treated *N. benthamiana* 16C of the first offspring generation (F1). The applied siRNA concentration was 14 ng µl-1.

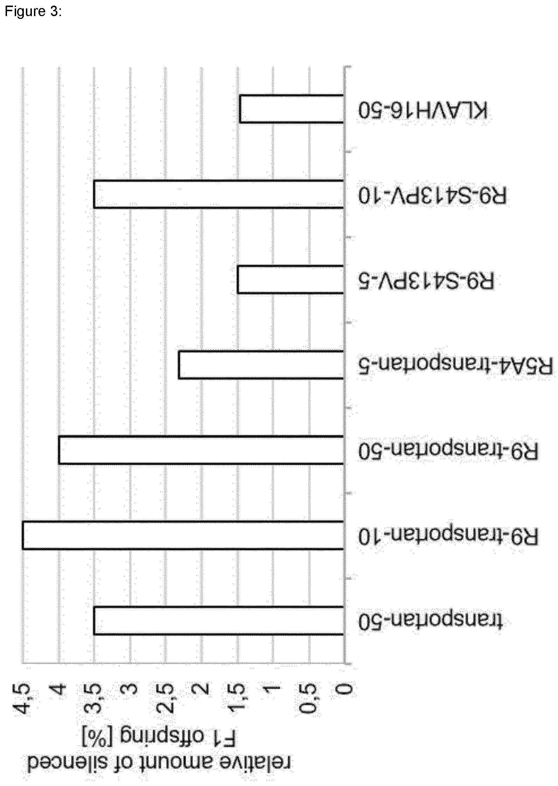

Figure 3: Relative amount of silenced offspring in the F1 generation with regard to the parental plants. Each line (sample) is derived from a single treated mother plant. Numbers at the end of the cell line name indicate the applied molar siRNA/CPP ratio (e.g. 50 = 50:1). Seeds were germinated on 0.5 x MS medium under sterile conditions, n=200.

### Example

### Treatment of plants with siRNA fusion polypeptide complexes

The fusion polypeptides according to SEQ ID NO.: 43 to 48 were subjected to a DNase I digestion for eliminating remaining DNA from the subsequent purification steps at 37°C for 15 minutes. The DNase I was deactivated for 10 minutes at 65°C. Thereafter, the siRNA was brought in contact with the fusion polypeptide sample. The siRNA concentration was 14 ng/µl and the molar ratios of siRNA to CPP carrying fusion polypeptide were 5:1, 10:1 and 50:1. They were mixed at 1100 rpm for 2 hours at room temperature in the presence of 0.1% Silwet^{®} L-77.

In one aspect, low pressure spraying (LPS) was used for plant treatment. This method involves spraying the siRNA:fusion polypeptide complex at 1.3 bar onto *N*. *benthamiana* in nanomolar amounts (ratio 1:10 siRNA:fusion polypeptide) per 100 to 500 µL buffer. The *N. benthamiana* plants express GFP transgenously, which is silenced by the introduced siRNA.

The plants were prepared by incubating them in a highly humid environment at a humidity of 60 to 100 % for one hour. The spraying of the leaves and/or the stems of the plant was performed using a Conrad^{™} HP-200 airbrush pistol. The application of the siRNA:fusion polypeptide mixture was performed with juvenile plants at an age of two to fourteen days after the end of the embryonal phase as subjects of the treatment. The juvenile plants were treated. The organs that were treated were the meristem, the leaves themselves and the leaf edges.

The siRNAs that were used to produce the results were RNA sequences having a length of 22 amino acids. The fusion polypeptides that were used were designed with the following cell penetrating peptides: transportan- (SEQ ID No.: 16), Rg-transportan (SEQ ID No.:17), R₅A₄-transportan (SEQ ID NO.: 18), S4₁₃PV (SEQ ID NO.:1), R₉- S4₁₃PV (SEQ ID NO.:2), and KLAVH₁₆(SEQ ID NO.:21). Accordingly, fusion polypeptides with SEQ ID NO.: 43 to 48 were used in the experiments.

The results were measured as GFP silencing ratio of all silenced leaves originating from the apical medical stem. Results were compared with the method of introducing siRNA to cells using carbon polymer dots (as described in Schwartz et al., 2020, "Carbon Dots for Efficient Small Interfering RNA Delivery and Gene Silencing in Plants").

In Figure 1, the results of the gene silencing experiments are displayed for the parental generation. The results of LPS treatment with transportan-, Rg-transportan-, R₅A₄-transportan-, S4₁₃PV-, R₉-S4₁₃PV- and KLAVH₁₆- 22nt siRNA complex in the molar RNA:CPP-fusion polypeptide ratios 5:1, 10:1, 50:1, as well as the overall result (combined) are shown. The results of the 10 % CPD (carbon polymer dots) -22nt siRNA complex control is shown under the bar 'combined' as only one fixed ratio was applied. The number of leaves evaluated depends on the individual and ranges from 1 to 6.

The first offspring generation (F1) of treated plants was also investigated for GFP silencing. It could be found that silencing was observed in cases where fusion polypeptides according to the invention designed with cell penetrating peptides were used to carry 22nt siRNA inside the target cells, silencing also occurred in the F1 generation (first offspring generation).The applied concentration of siRNA was 14 ng µl⁻¹.

The results are depicted in figure 2 and shows that the siRNA delivery with different fusion polypeptides according to the invention designed with cell penetrating peptides, several plants *(N. benthamiana)* exhibit silenced GFP levels also in the first offspring generation F1, while maintaining the seed production capacity. The relative amount of silenced plants in the F1 generation to the parental generation is shown in Figure 3. The numbers at the end of the sample names indicate the applied molar siRNA/CPP ratio (e.g. 50 = 50:1). Seeds were germinated on 0.5 x MS medium under sterile conditions with a sample size of 200.

## Claims

1. Fusion polypeptide comprising or consisting of at least three functional domains:
i) a substrate binding domain binding to a substrate non-covalently, wherein the substrate is selected from the group consisting of polypeptides, polysaccharides, DNA, RNA or a mixture of DNA and RNA;
ii) a cell penetrating domain;
iii) a signal sequence;
iv) optionally a linker domain; and
v) optionally a pH-sensitive autoprotease domain,
wherein the cell penetrating domain has a length of between 15 and 50 amino acid residues, preferably of 20 to 40 amino acid residues and especially preferably of between 24 to 34 amino acid residues, and
wherein the cell penetrating domain comprises at least 70 %, preferably at least 75 % and especially preferably at least 80 % amino acid residues with a helix potential value >1, dependent on the overall length of the cell penetrating domain.

2. Fusion polypeptide according to claim 1, wherein the cell penetrating domain comprises or consists of at least one amino acid sequence selected from the group consisting of SEQ ID NOs.: 1 to 24, or an amino acid sequence having a sequence identity of more than 90 %, preferably at least 91 %, preferably at least 92 %, preferably at least 93 %, preferably at least 94 %, preferably at least 95 %, preferably at least 96 %, preferably at least 97 %, preferably at least 98 % preferably at least 99 % to an amino acid sequence selected from the group consisting of SEQ ID NOs.: 1 to 24.

3. Fusion polypeptide according to claim 1 or 2, wherein the substrate binding domain comprises at least one sequence of the amino acid motif sequences IHYWN and VYVKV.

4. Fusion polypeptide according to any one of the preceding claims, wherein the substrate binding domain comprises or consist of an amino acid sequence having a length of between 200 and 600, preferably of between 250 and 550 amino acids.

5. Fusion polypeptide according to any one of the preceding claims, wherein the substrate binding domain comprises or consists of at least one amino acid sequence selected from the group consisting of SEQ ID NOs.: 25 and 26 or an amino acid sequence having at least 90 %, preferably at least 91 %, preferably at least 92 %, preferably at least 93 %, preferably at least 94 %, preferably at least 95 %, preferably at least 96 %, preferably at least 97 %, preferably at least 98 % preferably at least 99 % sequence identity to an amino acid sequence selected from the group consisting of SEQ ID NOs.: 25 and 26.

6. Fusion polypeptide according to any one of the preceding claims, wherein the signal sequence is an inclusion body promoting sequence or a secretion sequence, preferably a secretion sequence of secretion type IV or type II of gram negative bacteria.

7. Fusion polypeptide according to any one of the preceding claims, wherein the signal sequence comprises or consists of at least one amino acid sequence selected from the group consisting of SEQ ID NOs.: 27 and 28 or an amino acid sequence having at least 90 %, preferably at least 91 %, preferably at least 92 %, preferably at least 93 %, preferably at least 94 %, preferably at least 95 %, preferably at least 96 %, preferably at least 97 %, preferably at least 98 % preferably at least 99 % sequence identity to an amino acid sequence selected from the group consisting of SEQ ID NOs.: 27 and 28.

8. Fusion polypeptide according to any one of the preceding claims, wherein the fusion polypeptide comprises a linker domain and, wherein
the linker domain comprises an N-terminal alpha helix and/or a C-terminal sequence of a random coil structure, preferably wherein the linker domain comprises or consists of at least one amino acid sequence selected from the group consisting of SEQ ID NOs.:29 and 30 or an amino acid sequence having at least 90 %, preferably at least 91 %, preferably at least 92 %, preferably at least 93 %, preferably at least 94 %, preferably at least 95 %, preferably at least 96 %, preferably at least 97 %, preferably at least 98 % or preferably at least 99 % sequence identity to an amino acid sequence selected from the group consisting of SEQ ID NOs.:29 and 30.

9. Fusion polypeptide according to any one of the preceding claims, wherein the substrate to which the substrate binding domain binds non-covalently is DNA or RNA having a length of 3 to 3,000,000 base pairs, preferably a length of 3,000 to 30,000 base pairs, especially preferably a length of 30,000 to 300,000 base pairs.

10. Fusion polypeptide according to any one of the preceding claims, wherein the fusion polypeptide comprises or consists of an amino acid sequence selected from the group consisting of SEQ ID NOs.: 32 to 99 or an amino acid sequence having at least 90 %, preferably at least 91 %, preferably at least 92 %, preferably at least 93 %, preferably at least 94 %, preferably at least 95 %, preferably at least 96 %, preferably at least 97 %, preferably at least 98 % preferably at least 99 % sequence identity to an amino acid sequence selected from the group consisting of SEQ ID NOs.: 32 to 99.

11. Fusion polypeptide according to any one of the preceding claims, wherein the fusion polypeptide comprises or consists of the five functional domains i) to v),
preferably wherein the pH-sensitive autoprotease domain comprises or consists of at least one amino acid sequence according to SEQ ID No.: 31 or an amino acid sequence having at least 90 %, preferably at least 91 %, preferably at least 92 %, preferably at least 93 %, preferably at least 94 %, preferably at least 95 %, preferably at least 96 %, preferably at least 97 %, preferably at least 98 % preferably at least 99 % sequence identity to an amino acid sequence according to SEQ ID No.: 31.

12. Recombinant nucleic acid molecule encoding a fusion polypeptide according to any one of the preceding claims.

13. Genetically modified cell, including a recombinant nucleic acid molecule according to claim 12, wherein the cell is capable of expressing a fusion polypeptide according to any one of claims 1 to 11, preferably wherein the cell is selected from the group consisting of *Escherichia coli, Vibrio natrigens, Saccheromyces cerevisiae, Aspergillus niger,* green algae, microalgae, HEK T293 and Chinese hamster ovary cells (CHO).

14. Non-therapeutic use of a fusion polypeptide according to any one of the claims 1 to 11 for introducing a substance into a target cell, wherein the substance is selected from the group consisting of antibodies, small molecules, antibiotics, biologics, polypeptides, transcription factors or DNA, RNA or a mixture of DNA and RNA.

15. Non-therapeutic use of a fusion polypeptide according to claim 14, wherein the target cell is selected from the group consisting of plant cells, bacterial cells, mammalian cells and yeast cells, preferably plant cells.
